# EUROPEAN PATENT SPECIFICATION

(11) **EP 1 646 667 B1**
(45) Date of publication and mention of the grant of the patent: **10.10.2007**
(21) Application number: 04778035.8
(22) Date of filing: 09.07.2004
(51) Int. Cl.: C08F 255/02, C08J 3/22, D01F 8/04, A61L 15/16

(54) **METHOD FOR THE MANUFACTURE OF A FUNCTIONALISED POLYOLEFIN, FUNCTIONALISED POLYOLEFIN, BICOMPONENT FIBER, NONWOVEN AND HYGIENIC ABSORMENT PRODUCT**
VERFAHREN ZUR HERSTELLUNG EINES FUNKTIONALISIERTEN POLYOLEFINS, FUNKTIONALISIERTES POLYOLEFIN, ZWEIKOMPONENTENFASER, VLIESSTOFF UND HYGIENISCHES ABSORPTIONSPRODUKT
PROCEDE DE FABRICATION D'UNE POLYOLEFINE FONCTIONNALISEE, POLYOLEFINE FONCTIONNALISEE, FIBRE BICOMPOSEE, NON TISSE ET PRODUIT HYGIENIQUE D'ABSORPTION

(30) Priority: 11.07.2003 US 486526 P
(43) Date of publication of application: 19.04.2006
(73) Proprietor: DOW GLOBAL TECHNOLOGIES INC., Midland, Michigan 48674 (US)
(72) Inventor: ALLGEUER, Thomas, T., CH-8832 Wollerau (CH); KATZER, Karin, ZH/ZH-8810 Horgen (CH)
(74) Representative: Hayes, Adrian Chetwynd
(86) International application number: PCT/US2004/022325
(87) International publication number: WO 2005/007716

(56) References cited:
- WO-A-01/04200
- DE-A- 2 213 202
- US-A- 5 372 885
- US-B1- 6 331 595

## Description

The present invention relates to a method for the manufacture of a functionalised polyolefin by free radical grafting of a polyolefin with a monomer as well as to a bicomponent fiber and a nonwoven comprising the functionalised polyolefin.

Grafting of a monomer onto a polyolefin is a process known in the art. Grafting has generally been used to produce polyolefins having useful adhesive properties.

Grafted polyolefins and blends comprising them are generally useful for extrusion coating of metals, polymer films, paper, wood, glass. They are also useful as an adhesive layer in multi-layer structures, such as in the making of bicomponent fibers and in other applications where thermoplastic behavior, processability, tenacity and/or the adhesiveness are desired.

Fibers exhibiting enhanced adhesive and cohesive properties have been made of a blend of grafted polymers with one or more other polymers and are used as binder fibers. The blends can also be extruded as a sheath on a core of performance fibers to enhance the adhesive properties of the performance fiber. In such bicomponent fiber, the grafted polyolefins may be used to provide adhesion between the core and the sheath of the fiber and/or between the bicomponent fiber and other natural or synthetic fibers. Bicomponent fibers have found particular application in nonwoven fabrics prepared from performance fibers which would otherwise tend to separate easily in the fabric.

Conventionally, the grafting of a polyolefin is carried out in a polyolefin melt by a radical grafting reaction in the presence of a free radical initiator.

US-A-4,599,385 discloses a method for grafting copolymers wherein maleic acid or maleic anhydride is grafted to a poly(propylene-butene) backbone.

US-A-4,762,890 describes a method for grafting maleic anhydride to polyethylene in the presence of a peroxide as a free radical initiator.

Polyethylene undergoes cross-linking in the presence of heat and shear. Cross-linking is observed increased in the presence of peroxide. Products obtained by conventional grafting methods contain a significant amount of cross-linked polymer material and free monomer. A grafted polyolefin containing cross-linked polymer material or free monomer as obtained by conventional processes is undesirable in many applications.

In fiber spinning processes, cross-linked polymer material leads to breaks in the fiber. To remove cross-linked polymer material in the spinning line, a filter is used which has to be exchanged frequently. Productivity and spinning speed of the fiber spinning process is thus considerably reduced. Simultaneously, an amount of more than 0.1 wt percent of free monomer in the polymer causes fiber breaks, fume and smoke creation and heavy maintenance work.

US-A-6,331,595 describes a proposed method for reducing the cross-linked fraction in a product obtained by grafting a monomer onto a polyolefin in the presence of peroxide. According to said method the peroxide is introduced coated onto a carrier polymer.

The object of US-A-4,612,155 is to obtain a uniform product in the grafting of monomers onto polyolefins. It discloses a continuous process for the grafting of monomers onto homopolymers of ethylene or copolymers of ethylene and C₄-C₁₀ higher alpha-olefins in the presence of a second polymer. The second polymer is selected from a wide range of polymers.

Grafting levels of the products obtained by the known processes are unsatisfactory in many applications. In fiber applications, for example, the bond between fiber core and fiber sheath as well as between individual fibers is not sufficient which is particularly disadvantageous in the manufacturing of nonwovens.

A nonwoven material comprising bicomponent fibers is disclosed in US-A-5,981,410. The performance of conventional nonwovens is often unsatisfactory since a large portion of the fibers is not bonded to any other fiber or otherwise held in place by means of the structure formed by bonding of other fibers. These nonwoven material may exhibit poor abrasion resistance and low tensile strength.

In view of the deficiencies in the prior art, it remains advantageous to provide a method for the manufacture of functionalised polyolefins by grafting resulting in a product with a low amount of cross-linked polymer material and free monomer.

It also remains desirable to provide a method for the manufacture of functionalised polyolefins by grafting resulting in a product with high grafting levels.

In addition, it remains desirable to provide a bicomponent fiber having improved adhesion properties between fiber sheath and fiber core as well as between fibers and to provide a nonwoven material having a high tensile strength.

According to the present invention, a method is provided for the manufacture of a functionalised polyolefin by free radical grafting of a polyolefin with a monomer in the presence of 0.5 to 25 wt percent, based on the total weight of polyolefin and monomer, of a propylene polymer and/or copolymer which polypropylene polymer or copolymer has a free radical initiator distributed therein.

Due to the free radical initiator being distributed in the propylene polymer and/or copolymer the grafting reaction can be much better controlled. It has been found that the cross-linked fraction and the free monomer fraction of the functionalised polyolefin obtained according to the present invention is reduced, often significantly, and in some embodiments to an amount non-detectable by Soxhlett-extraction.

In addition, it has surprisingly been found that the graft level of the functionalised polyolefins obtained according to the present invention can be increased compared to the functionalised polyolefins obtained by more conventional methods.

Due to the reduced amount of the cross-linked fraction and of the free monomer fraction in the polymer product of the invention, fiber breaks are reduced during the spinning process and excellent fiber drawability is achieved.

Therefore, the problems of clogging of the filter in the spinning line and die pressure build-up is also reduced. This allows a longer stable run before changing the screen pack and the laborious process of changing and cleaning the screen pack is decreased.

Due to improved spinning process and the reduced maintenance of the spinning device, productivity of the spinning process is highly increased.

When the grafted polymer is used as a sheath material in a bi- or multi-component fiber, the higher grafting level of results in excellent bonding performance between fiber core and sheath and reduces core/sheath delamination. In addition, bonding performance between the individual fibers can be greatly improved.

As a result, when used in the production of nonwoven materials, dust generation is reduced. In addition, the waste resulting from the manufacturing process of nonwovens is reduced and maintenance of the production device simplified. This has the effect of improving the efficiency of the manufacturing process. In addition, tensile strength and abrasion resistance of the obtained nonwovens is enhanced resulting in a higher quality product.

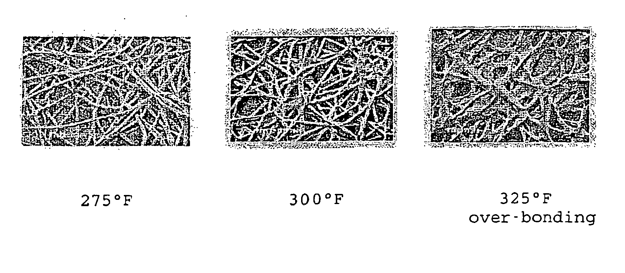

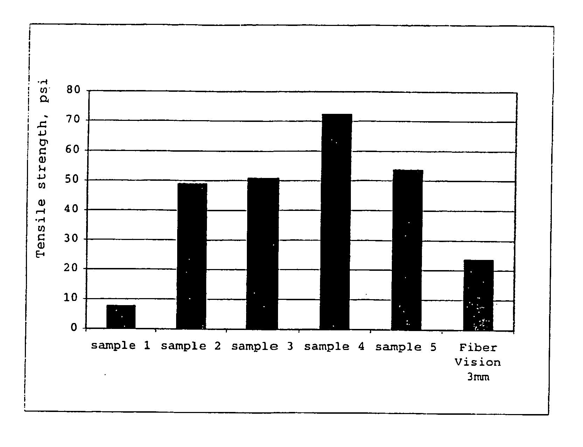

Figures 1 and 2 illustrate the results that can be obtained using the present invention with:
Figure 1 being microscopic pictures showing the effects of bonding; and
Figure 2 demonstrating the effect of grafted polymer on bonding strength;

Examples of polyolefin materials to be grafted that can be employed in the practice of the present invention include ethylene homopolymers and copolymers of ethylene with one or more C₄-C₁₀ α-olefins.

The monomer to be grafted to the polyolefin is preferably an ethylenically unsaturated compound comprising a carbonyl group that is conjugated with a double bond of the ethylenically unsaturated compound. Representatives of such compounds are carboxylic acids, anhydrides, esters and their salts, both metallic and non-metallic. Preferably, the olefinically unsaturated organic monomer is characterized by at least one ethylenic unsaturation conjugated with a carbonyl group. Preferred monomers are maleic acid, fumaric acid, acrylic acid, methacrylic acid, itaconic acid, crotonic acid, alpha-methyl crotonic acid and cinnamic acid and their anhydride, ester and salt derivatives as well as N-vinyl pyrrolidone. The monomer is most preferably selected from the group of maleic acid, maleic anhydride, acrylic acid and glycidyl methyacrylate. However other monomers, such as vinyl siloxanes may also be used according to this method.

The free radical initiator used in the practice of the present invention is preferably a peroxide. Examples of useful peroxides include aromatic diacyl peroxides, aliphatic diacyl peroxides, dibasic acid peroxides, ketone peroxides, alkyl peroxyesters, alkyl hydroperoxides, alkyl and dialkyl peroxides, such as diacetyl peroxide, 2,5-bis(t-butylperoxy)-2,5-dimethylhexane or 2,5-dimethyl-2,5-di(t-butylperoxy)hexyne-3.

Preferably, the propylene polymer employed in the practice of the present invention is a propylene homopolymer or a copolymer of propylene and a C₄-C₁₀ α-olefin.

In a preferred embodiment, the grafting process comprises the following steps:
melting a polyolefin, preferably in an extrusion apparatus, more preferably in a co-rotating twin-screw extruder, by heating and shearing (The polyolefin can be fed in pellet form to the extruder barrel);
adding to the polymer melt a propylene polymer and/or copolymer having a free radical initiator distributed therein and a monomer to be grafted, preferably into a polyolefin filled, pressurized section of the extruder.
maintaining this mixture at conditions sufficient to graft the polyolefin.

While the reaction conditions will depend on various factors, including the specific polyolefin being grafted, the monomer being grafted to the polyolefin, the polypropylene and free radical initiator, the grafting reaction is preferably conducted at a temperature from 190°C to 210°C for times of from 30 to 110 seconds.

Following the reaction, the reaction product may be devolatilized, preferably in one or more decompression sections of the extruder.

In the practice of the present invention, the free radical initiator is preferably homogeneously distributed in the propylene polymer and/or copolymer. Due to the homogenous distribution, the propylene polymer and/or copolymer and the free radical initiator are simultaneously contacted with the polyolefin to be grafted. While not being bond by theory, the homogeneous distribution of the peroxide is believed to lead to a more uniform reaction with a reduced cross-linked fraction. In addition, setting specific distribution patterns of free radical initiator in the propylene polymer and/or copolymer, a product having different properties may be obtained. In fact, the distribution of the free radical initiator can be established to assist in obtaining a grafted polymer of desired properties.

The amount of propylene polymer and free radical initiator used in the grafting reaction will depend on a variety of factors including the specific polyolefin being grafted, the conditions of grafting, and the desired amount of grafting. In general, the polypropylene polymer is used in a range of from 0.5 to 25, preferably from 1 to 10, weight percent where the weight percent is based on the total weight of polyolefin and monomer. The amount of free radical initiator is generally from 100 to 20,000, preferably from 500 to 10'000 parts per million based on the total weight of polyolefin and monomer.

The invention is preferably employed to obtain a functionalised polyolefin having a graft level of from 0.1 to 4, preferably from 0.2 to 3, percent. The melt index of the functionalised polyolefin preferably ranges from 0.1 to 500 g/10 min, preferably from 0.2 to 400 g/10 min.

The functionalised polyolefin obtained can be used as adhesive in multi-layer structures such as in cables, tubes, foils or films. The term "adhesive" covers any function of the product of the present invention in connection with adhesion, for example an adhesion promoter.

The functionalised polyolefins obtained are useful in fibers. Fiber spinning processes are well known in the art and involve extrusion of the fibers or filaments, cooling and drawing. Fiber spinning can be accomplished using a conventional melt spinning process (also known as "long spinning process"), with spinning and stretching being performed in two separate steps. Alternatively, a "short spinning process", which is a one step operation, may be used.

The functionalised polyolefin obtained according to the present invention is particularly useful for bicomponent fibers. Bicomponent fibers can be manufactured by contacting under thermally bonding conditions a core component comprising a thermoplastic polymer, primarily chosen for its mechanical properties or performance, and a sheath component comprising a functionalised polyolefin as obtained by the above described method. The bicomponent fibers can be manufactured by coextruding the core component and the sheath component using techniques well known in the art. The resulting fiber can have any cross-sectional configuration, for example a symmetrical or asymmetrical sheath/core or side-by-side configuration. The fibers produced can be used for melt-blown, spunbond or staple fiber applications.

In general and as mentioned, the core component is primarily selected on the basis of its mechanical properties or performance. For example, the core component can be chosen to provide stiffness in the bicomponent fiber. In most applications, the core material preferably comprises a polyester such as polyethylene terephtalate or polybutylene terephtalate, a polyolefin such as polypropylene, or a polyamide such as nylon.

The sheath component generally constitutes at least a part of the surface of the bicomponent fiber. The functionalised polyolefins obtained according to the method of the present invention can be used as the sheath component, either individually or as a component in a blend with a non-grafted olefinic polymer. When used in combination with a non-grafted polymer, the functionalised polymer is preferably used in an amount of from 1 to 30, more preferably from 2 to 25, weight percent based on the total weight of the blend.

The bicomponent fibers according to the present invention can be used in the manufacturing process of nonwovens, generally as so-called binder fibers. When used as binder fiber, a fiber mixture comprising the binder fibers and one or more natural and/or synthetic fibers such as polyether, polyamides cellulosics, modified cellulosics, wool or the like are thermally bonded to prepare the nonwoven.

The nonwoven can be used in filters, membranes, films, and hygienic absorbent products such as disposable diapers, feminine hygienic and adult incontinence products. In addition to the bicomponent fibers according to the present invention, the absorbent core of the absorbent products can comprise natural fibers such as cellulose fluff pulp fibres, synthetic fibers based on, for example polyolefin and/or polyester, and a superabsorbent polymer (SAP).

The bicomponent fibers may also be employed in conventional textile processing such as carding, sizing, weaving. Woven fabrics comprising the bicomponent fibers of the present invention may also be heat-treated to alter the properties of the resulting fabric.

The following examples are presented to illustrate the invention and should not be construed to limit its scope. All percentages and parts are by weight unless otherwise indicated.

### EXAMPLE 1

### Grafting of maleic acid anhydride (MAH) onto a polymer

Ninety-eight parts of a polymer as specified below, 2 parts of maleic acid anhydride (MAH), 2 parts of a polypropylene/peroxide masterbatch (available as PEROX PP 5, from Colux (Germany) having a content of 5 wt percent of 2,5-Dimethylene - 2,5 - di (tert-butyl peroxy) hexane based on the weight of the total masterbatch) and 0.1 part of a stabilizer (Irganox 1010 available from Ciba) was grafted in a Coperion Krupp Werner/Pfleiderer ZSK 25 at a screw speed of 150/min and a temperature of 180°C.

### The following polymers were used in the grafting process

Polymer A: A linear low density polyethylene made with a constrained geometry catalyst (Affinity* XU 58200.03 available from The Dow Chemical Company): 0.913 g/cm³; MFI at 190°C, 2.16kg: 30.
* Trademark of The Dow Chemical Company

Polymer B: A medium density linear low density polyethylene (ASPUN* 6806A available from The Dow Chemical Company): 0.930 g/cm³; MFI at 190°C, 2.16kg: 105.
* Trademark of The Dow Chemical Compan

Polymer C: A high density polymer (gas phase): 0.951 g/cm³ having a melt flow index at 190°C, 2.16kg: 40 (available as DMDA 8940 available from The Dow Chemical Company).

Polymer D: A high density polymer (gas phase): 0.952 g/cm³; MFI at 190°C, 2.16kg: 80 (available as DMDA 8980 available from The Dow Chemical Company).

Samples of 25 kilograms (kg) were prepared and analysed in terms of cross-linked fraction, graft level and free monomer content. The cross-linked fraction was determined by Soxhlett-extraction with xylene. The results were given in Table 1. A high density polyethylene having a density of 0.953 grams per cubic centimeter (g/cm³) and a melt flow index of 11 at 190°C, 2.16 kg was used as reference sample.

**Table 1**

| | cross-linked fraction, wt. percent | Graft level wt. percent | free MAH, wt. percent | Free MA, wt. percent |
|---|---|---|---|---|
| Control | 13.5 | 1 | 0.025 | 0.025 |
| Polymer C/MAH | 0.0 | 0.9 | 0.03 | 0.04 |
| Polymer A/MAH | 0.0 | 1.38 | <0.01 | 0.015 |
| Polymer D/MAH | 0.3 | 1.35 | 0.05 | 0.049 |
| Polymer B/MAH | 0.25 | 1.4 | < 0.01 | 0.0160 |

As shown in Table 1, the cross-linked fraction of the samples obtained by the method according to the present invention was significantly lower than the high density polymer control. In fact, using the test described, no cross-linked fraction was detected for samples of grafted Polymer C (DMDA 8940/MAH) and Polymer A. In addition, a significantly higher MAH graft level was detected for XU 58200.03/MAH and DMDA 8980/MAH compared to the reference sample.

### EXAMPLE 2

### Grafting of maleic acid anhydride (MAH) onto XU 58200.03 under varying compounding conditions

Ninety eight parts of XU 58200.03, two parts of maleic acid anhydride (MAH), two parts of an identical polypropylene/peroxide masterbatch as that used in Example 1 was premixed in a fast rotating mixing device. Extrusion was performed in a Theysohn TSK 30 co-rotating twin-screw extruder with a length/diameter ratio of 40D under varying gafting conditions.

The graft level and the amount of the insoluble (cross-linked) fraction in the product in relation to the following parameters were analysed:
residence time (tᵥ min, tᵥ max; evaluated by adding a color masterbatch)
mass temperature (T_{M}) at the die
screw speed (n)
extruder filler level (M)
mass flow (m')
The results were shown in Table 2. The screw configuration was comparable to the screw configuration of ZSK 25 as used in Example 1. Additional kneading elements were used for samples 17 to 22.

**Table 2**

| Sample Nr. | T_{M}/°C | n/(1/min) | tᵥ min.*/s | tᵥ max. **/s | M/percent | m'/kg/h | Graft level/wt percent | Insoluble Amount/wt percent |
|---|---|---|---|---|---|---|---|---|
| 1 | 190 | 100 | 45 | 85 | 50 | 20 | 0,95 | 0,00 |
| 2 | 190 | 100 | 45 | 85 | 60 | 25 | 1,38 | 0,61 |
| 3 | 190 | 100 | 45 | 85 | 80 | 40 | 1,18 | 0,00 |
| 4 | 190 | 100 | 45 | 85 | 85 | n.d.*** | n.d.*** | n.d.*** |
| 5 | 190 | 200 | 30 | 65 | 50 | 22 | 0,99 | 7,90 |
| 6 | 190 | 200 | 30 | 65 | 60 | 29 | 0,95 | 6,58 |
| 7 | 190 | 200 | 30 | 65 | 80 | 41 | 1,27 | 3,47 |
| 8 | 190 | 200 | 30 | 65 | 85 | 41 | 1,18 | 2,75 |
| 9 | 210 | 100 | 43 | 80 | 53 | 22 | 1,32 | 8,69 |
| 10 | 210 | 100 | 43 | 80 | 61 | 26 | 1,19 | 4,36 |
| 11 | 210 | 100 | 43 | 80 | 80 | 30 | 0,71 | 1,96 |
| 12 | 210 | 100 | 43 | 80 | 85 | n.d.*** | n.d.*** | n.d.*** |
| 13 | 210 | 200 | 31 | 65 | 50 | 22 | 0,80 | 0,00 |
| 14 | 210 | 200 | 31 | 65 | 60 | 30 | 0,87 | 0,00 |
| 15 | 210 | 200 | 31 | 65 | 80 | 42 | 1,02 | 0,00 |
| 16 | 210 | 200 | 31 | 65 | 85 | n.d.*** | n.d.*** | n.d.*** |
| 17 | 190 | 100 | 52 | 104 | 50 | 17 | 1,58 | 0,32 |
| 18 | 190 | 100 | 52 | 104 | 60 | 22 | 1,34 | 1,07 |
| 19 | 190 | 100 | 52 | 104 | 80 | 30 | 1,05 | 1,90 |
| 20 | 190 | 200 | 34 | 76 | 50 | 20 | 1,15 | 2,18 |
| 21 | 190 | 200 | 34 | 76 | 60 | 29 | 1,12 | 2,30 |
| 22 | 190 | 200 | 34 | 76 | 80 | 42 | 1,03 | 4,83 |

| | | | | | | | | |
|---|---|---|---|---|---|---|---|---|
| Where: * tᵥ min: the time after which the first colored polymer exits the extruder ** tᵥ max: the time after which the polymer turned to be colorless again *** not determined | | | | | | | | |

As shown by the results in Table 2, the conditions of the grafting reaction impact the results, with, in this instance, optimum performance, in terms of graft level and cross-linked fraction, obtained with the grafting conditions of sample 17.

### EXAMPLE 3

### Spinnability of bicomponent fibers in relation to the composition of the sheath component

The spinnability of bicomponent fibers identified in Table 3 were evaluated. In this Table, the Polymers A, B, C and Control were the same as the identically identified polymers in Example 1.

**Table 3**

| Sample No. | Core polymer | Sheath polymer prior to grafting | Grafted polymer applied to the sheath polymer |
|---|---|---|---|
| 1 | PP | XU 61800.34 | None |
| 2 | PP | XU 61800.34 | Control |
| 3 | PP | XU 61800.34 | Polymer A/MAH |
| 4 | PP | XU 61800.34 | Polymer C/MAH |
| 5 | PP | XU 61800.34 | Polymer B/MAH |

As noted in Table 3, in all samples of bicomponent fiber, the core polymer was polypropylene (Inspire* polypropylene designated H505-20Z available from The Dow Chemical Company with a melt flow rate of 20). The sheath for all samples was an linear low density polyethylene having a density of 0.953 g/cm³ and a melt flow index of 17 (190°C, 2.16 kg) available as Aspun* XU 61800.34 from The Dow Chemical Company grafted with the grafted with 10 weight percent of the grafted polymer based on the total weight of the sheath and grafted polymer.

The fiber spinning process was performed under the following spinning parameters:

### Spinning

spinneret: 2x175 holes = total 350 holes, 0.4mm
as-spun fiber denier target: 5.8 dpf (denier per filament)
number of die holes: 350 total (two packs of 175 holes)
die hole range: 0.4 mm
bicomponent layer ratio (sheath/core): 50/50
fiber geometry: round
spinning draw ratio: 1:1.02

The conditions for the fiber spinning of the bicomponent fibers were given in Table 4. The individual components and zones listed in Table 4 were well known to a person skilled in the art.

**Table 4**

| Condition | bicomponent fiber |
|---|---|
| Extruder A (Sheath): | |
| Metering Pump, rpm | 15.0-16.0 |
| Extruder Pressure, psi | 1100 |
| Temp, Zone 1, 2, 3, 4 (°C) | 200, 210, 215, 220 |
| | |
| Extruder B (Core): | |
| Metering Pump, rpm | 15.0-16.0 |
| Extruder Pressure, psi | 1100 |
| Temp, Zone 1, 2, 3, 4 (°C) | 215, 220, 230, 240 |
| | |
| Spin Head Temperature (°C) | 240 |
| A pump block (°C) | 240 |
| B pump block (°C) | 240 |
| Quench air temp (°C) | 10-15 |
| Spin finish speed, rpm | 20 |
| Finish type/level | 5550/E1.0 |
| Denier roll speed, m/min | 490 |
| Feed roll speed, m/min | 493 |
| Draw roll speed #1, m/min | 496 |
| Draw roll speed #2, m/min | 500 |
| Winder speed, m/min | 515 |
| Pack oven temp. (°C) | 245 |
| Output (g/hole/min) | 0.37 |

### Drawing

drawing draw ratio: 4.38:1
drawn denier target: 1.5 dpf (denier per filament)
staple cut length: 1/8 inch

The conditions for the drawing and the crimping of the bicomponent fibers were given in Table 5. The individual components listed in Table 5 were well known to a person skilled in the art.

**Table 5**

| drawing conditions: | | crimping conditions: | |
|---|---|---|---|
| No.1 draw rolls, m/min | 16.0 | Crimper roll, psi | 6 |
| Temp, roll #1, (°C) | off | Cheek plate, psi | 80-90 |
| Temp, roll#2 (°C) | 50 | Flapper, psi | Set |
| Temp, roll#3 (°C) | 55 | Steam at crimper | 4-5 |
| Temp, roll #4 (°C) | 65 | Dryer temp.,°C | 105 |
| Temp, roll#5 (°C) | 70 | Dryer dwell time Hz | 4.5 |
| Nip roll | yes | Dryer fan speed, Hz | 25 |
| Water bath temp., °C | 110 | Lubricant level, percent | 0.2-0.4 |
| No.2 draw rolls. M/min | 45 | Denier/ filament | 1.5-1.7 |
| Temp, roll#1 (°C) | 60 | Staple length | 1/8" |
| Temp, roll#2 (°C) | 60 | Crimp level/Angle | 6-8/105-120 |
| Temp, roll#3 (°C) | 65 | Pounds produced | 25 |
| Temp, roll #4 (°C) | 70 | | |
| Temp, roll#5 (°C) | 75 | | |
| Nip roll | yes | | |
| Steam tube | On | | |
| No.3 draw rolls. M/min | 70 | | |
| Tension stand, lbs | 35-40 | | |
| Kiss roll, m/min w/ lube | 22 | | |

Draw ratio was defined as the #2 draw roll speed (constant at 500 meters per minute (m/min) divided by the denier roll speed (constant at 490 m/min), which equals 1:1.02. The drawing was carried out in the second stage of the fabrication in which the filament packages (total of 86 for the samples; 2 minute spinning packages) were drawn, crimped, dried, and cut for airlaid trials. Approximately 25 pounds (1.13 kg) crimped staple fiber of each sample was collected.

The samples of bicomponent fibers according to Table 3 were evaluated in terms of elongation, tenacity, staple fiber dpf (denier per filament), staple length and processability in the airlaid process. The results were given in Table 6.

**Table 6**

| **Sample No.** | **dpf** | **Tenacity g/den** | **Elongation at break, percent** | **Staple length** |
|---|---|---|---|---|
| 1 | 1.58 | 3.03 | 80.0 | 0.122 |
| 3 | 1.60 | 3.45 | 69.4 | 0.125 |
| 4 | 1.59 | 3.68 | 62.0 | 0.125 |
| 5 | 1.48 | 3.40 | 87.0 | 0.125 |

Sample No. 1 whose sheath component does not comprise a sheath additive shows a tenacity that was much lower than the one measured for the bicomponent fibers according to the present invention.

### EXAMPLE 4

### Air-laid trials

In order to evaluate the dispersion of the bicomponent binder fibers in airlaid trials, the samples described in Table 3 were prepared containing orange pigment. The air-laid forming trials were conducted on a Dan-Web airlaid pilot plant machine. Each sample of the bicomponent fibers was mixed with 12 weight percent of cellulose fluff pulp based on the total weight of the binder fibers. The nonwoven material was prepared having 100 grams per square mete (g/m²) cores/pads.

Following the fabrication of the airlaid cores/pads, the cores/pads were heated in a platen press for up to 30 seconds at 275, 300, 325 and 350°F (135°C, 149°C, 163°C and 177°C) to evaluate the bonding window and to bond the binder fibers to the cellulose pulp. Following this step, ten tensile specimens were cut out from each core/pad (and each of the above fiber compositions) and tested in an Instron machine at 0.5"/min testing speed. The dry tensile strength (or binding strength) of each air-laid nonwoven from the above tests was measured. In addition, microscopic pictures were prepared to evaluate the bonding characteristics of the various fiber compositions.

### Evaluation of bonding window

The evaluation of the bonding window of the binder fibers in the range of from 275 °F (135°C) to 350°F (177°C) shows that if a too low bonding temperature may result in under-bonding of the air-laid nonwoven (low dry tensile strength). Alternatively, a too high bonding temperature may result in over-bonding, meaning the binder fiber completely melts and loses its characteristics.

The microscopic pictures as shown in Fig. 1 present part of the analysis, including examples for under- and over-bonding. In this example, the bicomponent fiber provides melting characteristics at 275°F (135°C) with good bonding observed at 300°F (149°C), and over-bonding being shown at 325°F (163°C).

### Evaluation of dry tensile strength

To evaluate the bonding performance (dry tensile strength or binding strength) of the nonwoven comprising bicomponent fibers according to Table 3, the tensile strength was measured on the fabricated air-laid nonwoven bonded at 300°F (149°C). The results of this evaluation were shown in Figures 2 and 3. Also included in the analysis was the data collected on Fibervision binder fibers that were bonded and tested under the same conditions.

Fig. 2 demonstrates that the addition of a grafted polymer can have a considerable effect on the binding strength of the air-laid nonwoven. Specifically, significant improvements in terms of the dry tensile strength of the bicomponent fibers of the present invention compared to Sample 1 and the commercially available fibers supplied by Fibervision were shown.

The amount of grafting has an impact on the tensile strength of the corresponding nonwoven material. Specifically, the tensile strength of Sample 1 was less than 10 pounds per square inch (psi) while the nonwoven materials prepared from the grafted polymer all exhibited an average tensile strength of greater than 45 psi. The average tensile strength of Sample 2 was 49 psi, Sample 3 was 50 psi and Sample 5 is 53 psi). Sample no. 4 exhibits a tensile strength of greater than 70 psi. Thus, in this example, the highest tensile strength of the nonwoven was detected at a graft concentration of 0.9 wt percent (sample no. 4).

## Claims

1. A method for the manufacture of a functionalised polyolefin by free radical grafting of a polyolefin with a monomer in the presence of 0.5 to 25 weight percent, based on the total weight of polyolefin and monomer, of a propylene polymer and/or copolymer which propylene polymer or copolymer comprises a free radical initiator distributed therein.

2. A method according to claim 1 wherein the polyolefin is an ethylene homopolymer.

3. A method according to claim 1 wherein the polyolefin is an ethylene/C₄-C₁₀ α-olefin copolymer.

4. A method according to claim 1 wherein the propylene polymer is a propylene homopolymer.

5. A method according to claim 4 wherein the propylene copolymer is a propylene/C₄-C₁₀ α-olefin copolymer.

6. A method according to Claim 1 wherein the free radical initiator is homogenously distributed in the propylene polymer and/or copolymer.

7. A method according to Claim 6 where the free radical initiator is a peroxide used in an amount of from 500 to 10,000 weight parts per million parts of polyolefin and monomer.

8. A method according to Claim 1 wherein the monomer is an ethylenically unsaturated compound comprising a carbonyl group which is conjugated with a double bond of the ethylenically unsaturated compound.

9. A method according to claim 8 wherein the monomer is selected from the group of maleic acid, maleic anhydride, acrylic acid and glycidyl methacrylate.

10. A functionalised polyolefin obtained by the method according to Claim 1.

11. A functionalised polyolefin according to claim 10 having a graft level from 0.1 to 4 percent by weight.

12. A bicomponent fiber comprising a core component and a sheath component, said sheath component comprising a functionalised polyolefin of Claim 10.

13. A bicomponent fiber according to claim 12, wherein the core component comprises a polyester, a polyolefin and/or a polyamide.

14. The bicomponent fiber according to claim 12 wherein the core component is selected from the group consisting of a polyester, a polyolefin, a polyamide, or combinations thereof.

15. A bicomponent fiber according to claim 13 wherein the sheath component comprises a blend of the functionalised polyolefin of Claim 10.

16. A bicomponent fiber according to claim 15, the sheath component comprising 1 to 30 weight percent, based on the total weight of the blend, of the functionalised polyolefin.

17. A nonwoven comprising a functionalised polyolefin of Claim 10.

18. A nonwoven comprising a bicomponent fiber of Claim 12.

19. A hygienic absorbent product comprising the nonwoven of Claim 18.

## Patentansprüche

1. Verfahren zur Herstellung eines funktionalisierten Polyolefins durch freies radikalisches Pfropfen eines Polyolefins mit einem Monomer in der Präsenz von 0,5 bis 25 Gewichtsprozent, basierend auf dem Gesamtgewicht von Polyolefin und Monomer, eines Propylenpolymers und/oder -copolymers, welches Propylenpolymer oder - copolymer einen freien radikalischen Initiator umfasst, der in demselben verteilt ist.

2. Verfahren nach Anspruch 1, wobei das Polyolefin ein Ethylenhomopolymer ist.

3. Verfahren nach Anspruch 1, wobei das Polyolefin ein Ethylen/C₄-C₁₀ α-Olefin-Copolymer ist.

4. Verfahren nach Anspruch 1, wobei das Propylenpolymer ein Propylenhomopolymer ist.

5. Verfahren nach Anspruch 4, wobei das Propylencopolymer ein Propylen/C₄-C₁₀ α-Olefin-Copolymer ist.

6. Verfahren nach Anspruch 1, wobei der freie radikalische Initiator homogen in dem Propylenpolymer und/oder -copolymer verteilt ist.

7. Verfahren nach Anspruch 6, wobei der freie radikalische Initiator ein Peroxid ist, das in einer Menge von 500 bis 10000 Gewichtsteilen pro Million Teile von Polyolefin und Monomer verwendet wird.

8. Verfahren nach Anspruch 1, wobei das Monomer eine ethylenisch ungesättigte Verbindung ist, die eine Carbonylgruppe umfasst, die mit einer Doppelbindung der ethylenisch ungesättigten Verbindung konjugiert ist.

9. Verfahren nach Anspruch 8, wobei das Monomer aus jener Gruppe gewählt wird, die aus Maleinsäure, Maleinsäureanhydrid, Acrylsäure und Glycidylmethacrylat besteht.

10. Funktionalisiertes Polyolefin, welches durch das Verfahren nach Anspruch 1 gewonnen wird.

11. Funktionalisiertes Polyolefin nach Anspruch 10, das einen Pfropfgrad von 0,1 bis 4 Gewichtsprozent aufweist.

12. Zweikomponentenfaser, umfassend eine Kernkomponente und eine Mantelkomponente, wobei die Mantelkomponente ein funktionalisiertes Polyolefin aus Anspruch 10 umfasst.

13. Zweikomponentenfaser nach Anspruch 12, wobei die Kernkomponente einen Polyester, ein Polyolefin und/oder ein Polyamid umfasst.

14. Zweikomponentenfaser nach Anspruch 12, wobei die Kernkomponente aus jener Gruppe gewählt wird, die aus einem Polyester, einem Polyolefin, einem Polyamid oder aus Kombinationen derselben besteht.

15. Zweikomponentenfaser nach Anspruch 13, wobei die Mantelkomponente einen Blend des funktionalisierten Polyolefins aus Anspruch 10 umfasst.

16. Zweikomponentenfaser nach Anspruch 15, wobei die Mantelkomponente 1 bis 30 Gewichtsprozent, basierend auf dem Gesamtgewicht des Blends, des funktionalisierten Polyolefins umfasst.

17. Vliesstoff, umfassend ein funktionalisiertes Polyolefin aus Anspruch 10.

18. Vliesstoff, umfassend eine Zweikomponentenfaser aus Anspruch 12.

19. Hygienisches Absorptionsprodukt, umfassend den Vliesstoff aus Anspruch 18.

## Revendications

1. Procédé de préparation d'une polyoléfine fonctionnalisée, par greffage radicalaire d'un monomère sur une polyoléfine, en présence de 0,5 à 25 % en poids, par rapport au poids total de la polyoléfine et du monomère, d'un polymére et/ou d'un copolymère de propylène, lequel polymère ou copolymère de propylène comprend un amorceur radicalaire, réparti en son sein.

2. Procédé conforme à la revendication 1, dans lequel la polyoléfine est un homopolymère d'éthylène.

3. Procédé conforme à la revendication 1, dans lequel la polyoléfine est un copolymère d'éthylène et d'α-oléfine en C₄₋₁₀.

4. Procédé conforme à la revendication 1, dans lequel le polymère de propylène est un homopolymère de propylène.

5. Procédé conforme à la revendication 4, dans lequel le copolymère de propylène est un copolymère de propylène et d'α-oléfine en C₄₋₁₀.

6. Procédé conforme à la revendication 1, dans lequel l'amorceur radicalaire est réparti de manière homogène au sein du polymère et/ou du copolymère de propylène.

7. Procédé conforme à la revendication 6, dans lequel l'amorceur radicalaire est un peroxyde qu'on emploie à raison de 500 à 10000 parties en poids par million de parties de polyoléfine et de monomère.

8. Procédé conforme à la revendication 1, dans lequel le monomère est un composé à insaturation éthylénique, qui comporte un groupe carbonyle conjugué avec une double liaison du composé à insaturation éthylénique.

9. Procédé conforme à la revendication 8, dans lequel le monomère est choisi dans l'ensemble formé par l'acide maléique, l'anhydride maléique, l'acide acrylique et le méthacrylate de glycidyle.

10. Polyoléfine fonctionnalisée, obtenue par un procédé conforme à la revendication 1.

11. Polyoléfine fonctionnalisée, conforme à la revendication 10, dont le taux de greffage vaut de 0,1 à 4 % en poids.

12. Fibre bicomposant, comprenant un composant de coeur et un composant de gaine, lequel composant de gaine comprend une polyoléfine fonctionnalisée conforme à la revendication 10.

13. Fibre bicomposant, conforme à la revendication 12, dans laquelle le composant de coeur comprend un polyester, une polyoléfine et/ou un polyamide.

14. Fibre bicomposant, conforme à la revendication 12, dans laquelle le composant de coeur est choisi dans l'ensemble constitué par les polyesters, polyoléfines, polyamides, et combinaisons de tels polymères.

15. Fibre bicomposant, conforme à la revendication 13, dans laquelle le composant de gaine comprend un mélange d'une polyoléfine fonctionnalisée conforme à la revendication 10.

16. Fibre bicomposant, conforme à la revendication 15, dans laquelle le composant de gaine comprend 1 à 30 %, en poids rapporté au poids total du mélange, de ladite polyoléfine fonctionnalisée.

17. Non-tissé comprenant une polyoléfine fonctionnalisée conforme à la revendication 10.

18. Non-tissé comprenant une fibre bicomposant conforme à la revendication 12.

19. Produit absorbant hygiénique, comprenant un non-tissé conforme à la revendication 18.
